# EUROPEAN PATENT SPECIFICATION

(11) **EP 4 356 883 B1**
(45) Date of publication and mention of the grant of the patent: **18.02.2026**
(21) Application number: 23180519.3
(22) Date of filing: 21.06.2023
(51) Int. Cl.: A61F 9/04, H04R 5/033

(54) **EYE MASK**
AUGENMASKE
MASQUE OCULAIRE

(30) Priority: 17.10.2022 US 202218047131; 19.10.2022 US 202217969312
(43) Date of publication of application: 24.04.2024
(73) Proprietor: Manta Sleep LLC, Jackson, WY 83001 (US)
(72) Inventor: SCHWARZ, Benjamin, Jackson, WY, 83001 (US); ZHANG, Mark, Jackson, WY, 83001 (US)
(74) Representative: Cabinet Chaillot

(56) References cited:
- WO-A1-2018/081834
- US-A1- 2017 264 994
- US-A1- 2019 110 927
- US-A1- 2019 149 903
- US-B1- 11 207 490
- US-B2- 8 213 670

## Description

### 1. Field of the Invention

The present disclosure relates to an article for daily use, especially to an eye mask that shades light.

### 2. Description of the Prior Arts

An eye mask is a daily necessity for helping people to sleep. If the brightness of the environment is high, it will easily affect the sleep quality and even make many people struggle to fall asleep. Therefore, an eye mask worn during sleep for shading light from the environment becomes an important apparatus to maintain the sleep quality.

Moreover, noise in the environment also bothers people during sleep. Thus, many people like to sleep with music or white noise, which blocks the noise from the environment and provides another way to help them fall asleep. As a result, some eye masks include two speakers to cover the user's ears. Because the structure of the eye mask is fixed but the head size differs from person to person, the speakers may not exactly cover the ears. Thus, the environment noise is still capable of entering the user's ears. Accordingly, a need remains for improved sleep masks having speaker and audio functionality.

Besides, an eye mask may have two surfaces, one of the surfaces is configured to contact the user's skin, and the other is configured to shade the light. The surface configured to contact skin may be tender and washable, but the surface configured to shade the light may become fragile during washing or form a pattern that will fade after washing. Often, people are thus resistant to washing the eye mask.

However, people often sweat in sleep, so the eye mask that contacts the skin may absorb sweat continuously. After a period of time of use, the user may feel itchy or uncomfortable when wearing the eye mask and then have to wash it.

If the conventional eye mask includes one or more speakers, since the speakers are made of electric components, the eye mask with one or more speakers is generally not washable. If the user cannot tolerate an uncomfortable eye mask, but is resistant or cannot clean it, a new eye mask is needed but this is expensive. Accordingly, a need remains for improved sleep masks having speakers.

In addition, conventional eye mask is disclosed by PCT Publication NO. 2018081834 A1, US Publication NO. 2019/0149903 A1, US Patent NO. 11,207,490 B1, US Publication NO. 2019/0110927 A1, US Patent NO. 8,213,670 B2, and US Publication NO. 2017/264994 A1.

The main objective of the present invention is to provide an eye mask with one or more movable speakers configured for noise blocking, and the eye mask includes multi-piece so that some pieces are washable

The eye mask has a mask main body and at least one speaker assembly. The mask main body is configured to cover a user's eyes and ears. The mask main body includes at least one inner space and at least one slot. The at least one inner space corresponds to the user's ears in location. The at least one slot is formed to allow access to the at least one inner space. The at least one speaker assembly is mounted in the at least one inner space and configured to be adjustable and movable in the at least one inner space. Each one of the at least one speaker assembly includes a speaker and an operation unit. The speaker is movably positioned in the at least one inner space of the mask main body. The operation unit is located out of the mask main body and connected to the speaker via the at least one slot, which allows the user to manually adjust or manipulate the operation unit to slide or move the speaker. The operation unit includes a tab. The tab has two ends, and one of the two ends is mounted on the speaker and the other end extends out of the at least one slot.

With the aforementioned structures, the eye mask of the present disclosure is capable of not only shading light but also substantially reducing or blocking noise. When wearing the present eye mask, the user may cover the eyes and connect and tie two ends of the second headband, thereby securing the eye mask on the head. The eye mask also substantially covers the user's ears. Then, the two speaker assemblies may be moved or adjusted along the two slots of the first headband so that the two speaker assemblies can align with the user's ears respectively. As a result, when the user desires some assistance to fall asleep, the two speaker assemblies can provide a desired sound, music, or white noise to the user's ears, which can reduce or substantially block surrounding or environment noise and thus helps the user to sleep.

Other objectives, advantages and novel features of the invention will become more apparent from the following detailed description when taken in conjunction with the accompanying drawings.

### IN THE DRAWINGS

Fig. 1 is a front view of an eye mask in accordance with the present disclosure;
Fig. 2 is a rear view of the eye mask of Fig. 1;
Fig. 3 is a top view of the eye mask of Fig. 1;
Fig. 4 is a front view of a first headband of the eye mask of Fig. 1;
Fig. 5 is a rear view of the first headband of the eye mask of Fig. 4;
Fig. 6 is a front view of a second headband of the eye mask of Fig. 1;
Fig. 7 is a rear view of the second headband of the eye mask of Fig. 6;
Fig. 8 is an exploded view of a first headband of the eye mask in Fig. 1;
Fig. 9 is a sectional view of an inner space and a speaker assembly of the eye mask in Fig. 1;
Fig. 10 is an enlarged view of a slot and a speaker assembly of the eye mask in Fig. 1;
Fig. 11 is an enlarged view of a slot of the eye mask in another configuration of the present disclosure;
Fig. 12 is an enlarged view of a slot of the eye mask in another configuration of the present disclosure;
Fig. 13 is a front view of an eye cup of the eye mask of Fig. 1;
Fig. 14 is a rear view of the eye cup of the eye mask of Fig. 13;
Fig. 15 is an enlarged view of a speaker assembly of the eye mask in Fig. 8;

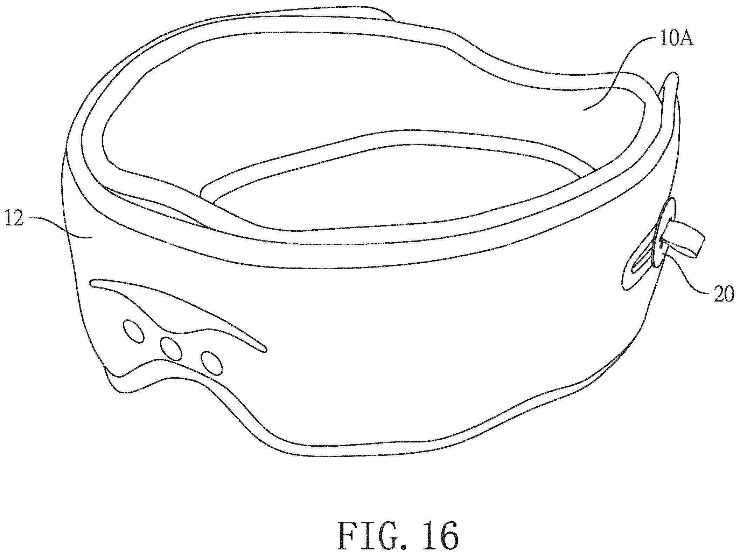

Fig. 16 is a perspective view of an eye mask in accordance with another embodiment of the present disclosure; and

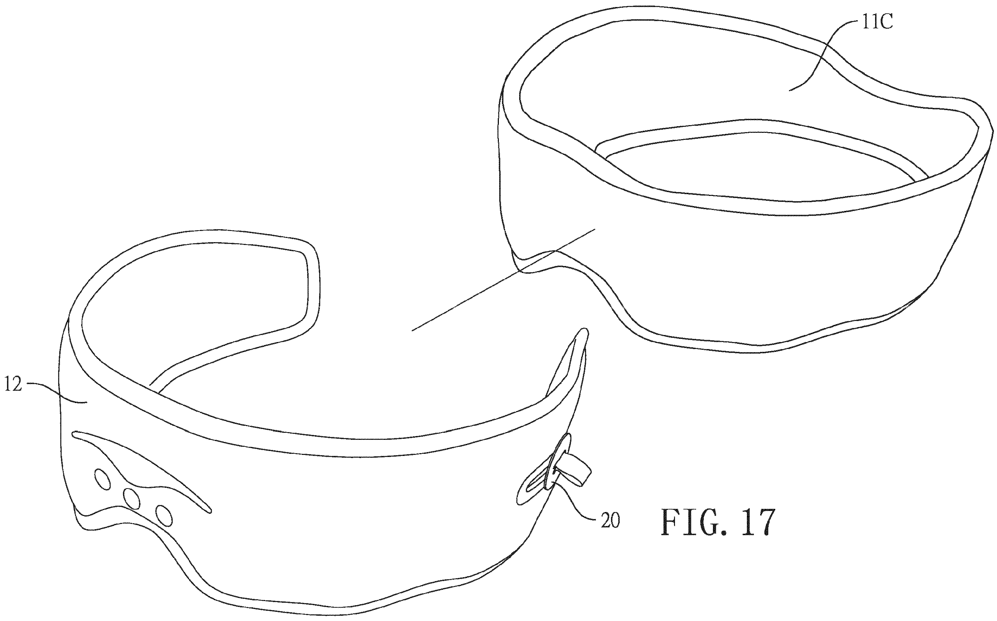

Fig. 17 is an exploded view of the eye mask of Fig. 16.

### DETAILED DESCRIPTION OF THE PREFERRED EMBODIMENTS

The invention relates to an eye mask and is defined by the appended claims.

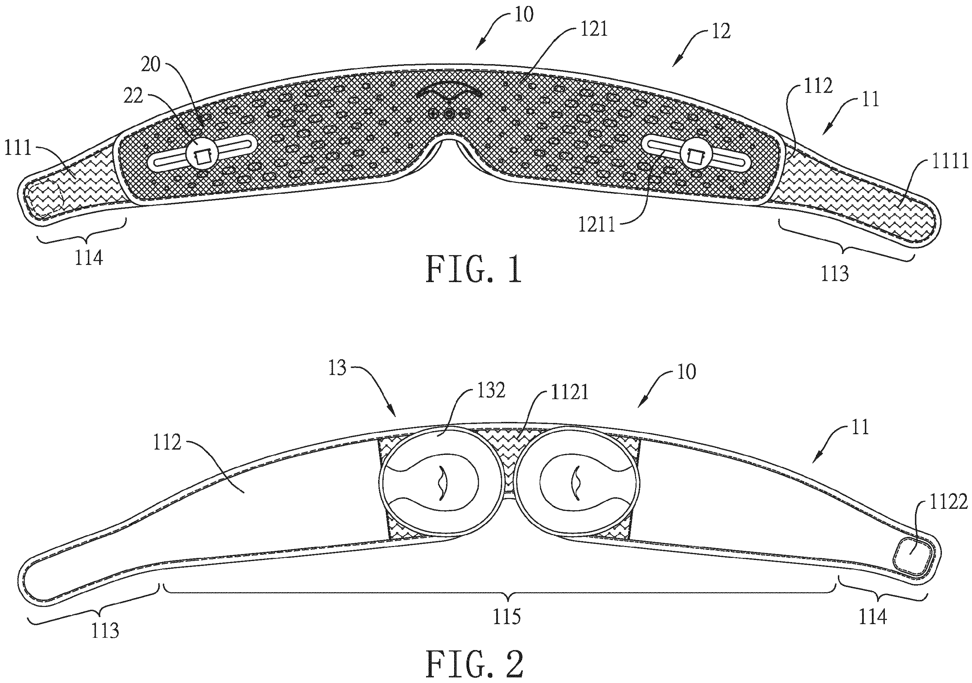

Please refer to Fig. 1 to Fig. 3. An eye mask provided in accordance with the present disclosure includes a mask main body 10 and two speaker assemblies 20.

The mask main body 10 is configured to cover a user's eyes and ears and thereby shades light and noise. The mask main body 10 may only include one headband, such that said headband in use is configured to cover the user's eyes and ears and receive the two speaker assemblies 20. The mask main body 10 may include two headbands configured to compliment and engage with each other via a reusable adhesive.

In this example, the mask main body 10 includes a first headband 11 and a second headband 12. The first headband 11 is configured to contact the user, and the second headband 12 is configured to receive the two speaker assemblies 20. In this embodiment, the mask main body 10 further includes two eye cups 13.

The first headband 11 can be made of fabric, so the first headband 11 is comfortable and breathable and is capable of absorbing sweat. Moreover, the first headband 11 is configured to be washable. According to the present disclosure, being washable means that the first headband 11 will generally stay intact under standard washing conditions which may include being washed in a laundry machine or being hand washed. In this example, the first headband 11 does not include any electronic components which could be damaged or destroyed if exposed to excessive water or normal washing/laundry conditions. Moreover, the first headband 11 can include an internal foam material covered by fabric to provide added comfort. The internal foam material should also be configured to be washable.

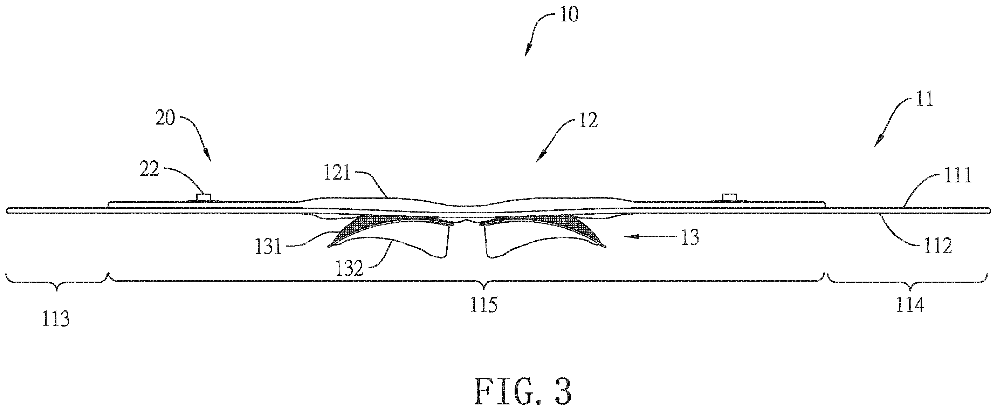

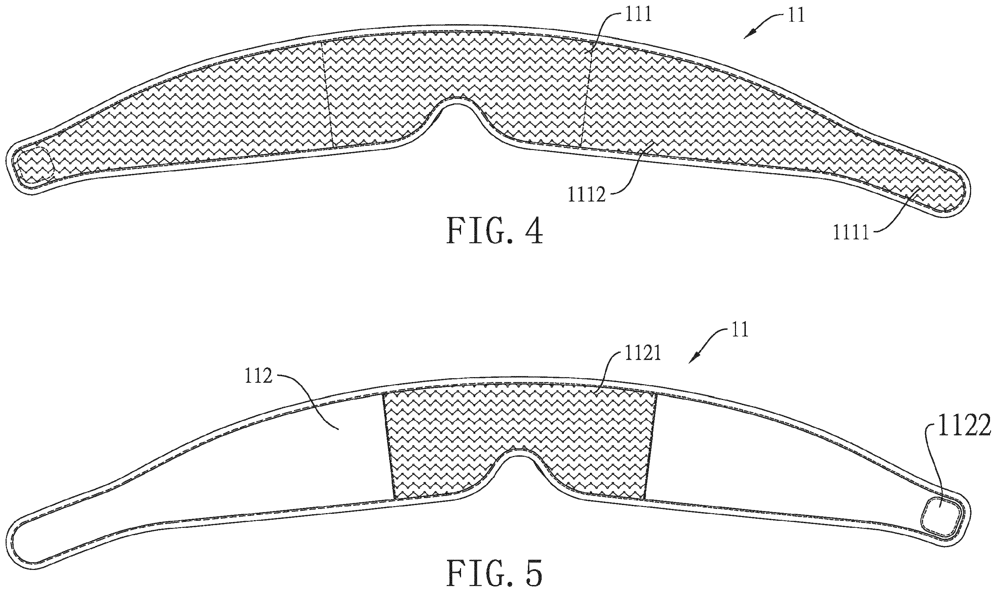

Please refer to Figs. 3 to 5. The first headband 11 defines a front surface 111 and a rear surface 112, a first end 113 and an opposite second end 114, and a middle section 115 between the first end 113 and the second end 114. The front surface 111 and the rear surface 112 are opposite each other. The rear surface 112 is configured to cover a user's eyes and ears. The first end 113 and the second end 114 are opposite two ends of the first headband 11. The first headband 11 can be curved and the middle section 115 can be configured to be higher than the first end 113 and the second end 114 when worn by the user, and thereby the user may have better wearing experience. In this embodiment, the first end 113 is longer than the second end 114.

In an example, a length of the first headband 11 is larger than the circumference of the user's head, such that when the two ends of the first headband 11 are secured on each other and the first headband 11 forms a loop. The first headband 11 includes an end fluff layer 1111, a front fluff layer 1112, and a rear fluff layer 1121. The end fluff layer 1111 is formed on the front surface 111 of the first end 113. The front fluff layer 1112 is formed on the front surface 111 of the middle section 115. The end fluff layer 1111 and the front fluff layer 1112 may be connected to each other and formed integrally. The rear fluff layer 1121 is formed on the rear surface 112 of the middle section 115. In this example, the rear fluff layer 1121 extends only a portion of the rear surface 112 and corresponds to an area designed to cover the eyes of a user or to host eye cups 13. The eye cups 13 can also include a fastener feature to detachably attach to the first headband 11.

The first headband 11 includes an attach pad 1122. The attach pad 1122 is provided on the rear surface 112 of the second end 114. The attach pad 1122 is configured to detachably connect to the end fluff layer 1111, such that the second end 114 is secured on the first end 113, thereby the first headband 11 forming the loop. Thus, a user's head may be enveloped in the first headband 11 and secured in place for using during sleep and centering the eye cups 13 or the middle section 115 substantially around the eyes of the user. Attach pad 1122 can include an adhesive surface for detachably securing the second end 114. An adhesive surface may include a fastener like a hook-and-loop fastener, commonly referred to as VELCRO. Each end can be include a corresponding fastener strip that can be attached via any attachment means like glue, sewing, or otherwise.

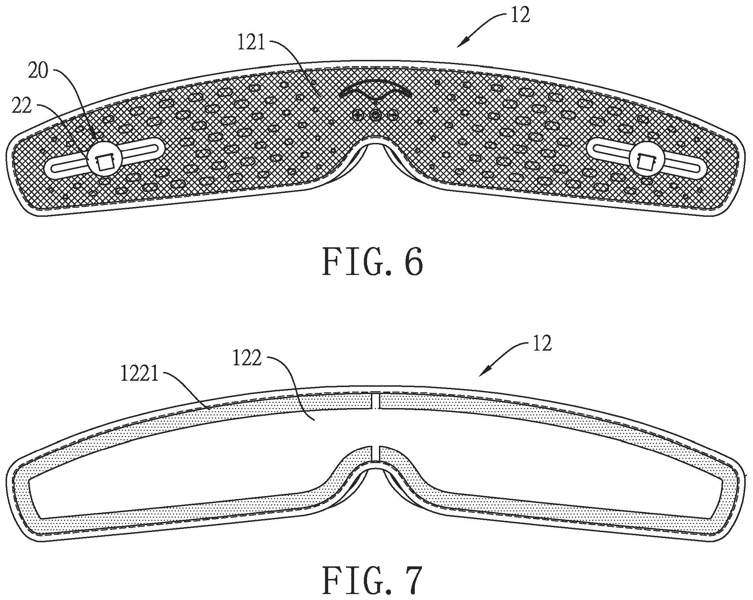

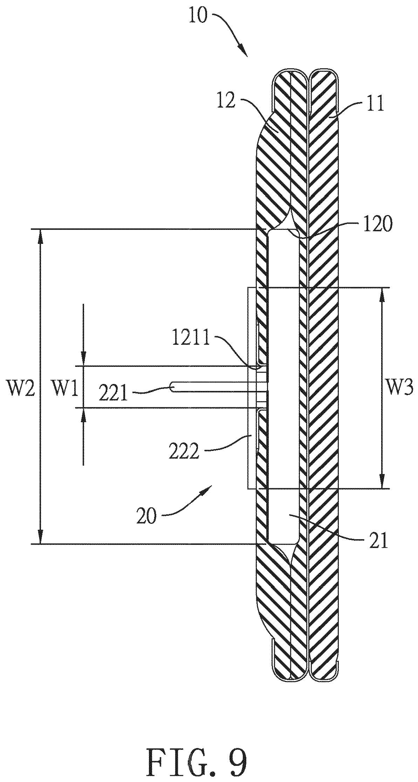

Please refer to Figs. 6 to 10. The second headband 12 is configured to be detachably mount on the first headband 11 and configured to contact a user's skin. A length of the second headband 12 is larger than a length from an ear to another ear along a circumference of the user's head. The two speaker assemblies 20 are provided and secured in the second headband 12 and correspond to the user's ears in location. Typically, the second headband 12 is not washable since it contains electronic components (e.g., the speaker assemblies 20).

The detachability allows for customized positioning which adds to the comfort and individualization of each headband. Therefore, the first headband 11 is located between the eye cups 13 and the second headband 12.

The second headband 12 includes a front surface 121 and a rear surface 122. The rear surface 122 of the second headband 12 faces the user and includes an attaching portion 1221. The attaching portion 1221 is provided on the rear surface 122 of the second headband 12 and configured to detachably attach onto the front fluff layer 1112 of the first headband 11, such that the second headband 12 is detachably secured on the first headband 11. The attaching portion 1221 may be composed of at least one attaching strip, and the attaching strip is provided on the second headband 12 and extends along a contour of the second headband 12. The attachment portion may include an adhesive surface like a fastener like a hook-and-loop fastener, commonly referred to as VELCRO. Each end can include a corresponding fastener strip that can be attached via any attachment means like glue, sewing, or otherwise.

The second headband 12 defines at least two inner spaces 120 positioned opposite from each other and at least one slot 1211. Precisely, the second headband 12 includes a first sheet and a second sheet, and at least two inner spaces 113 formed between the first sheet and the second sheet. The two inner spaces 120 correspond to the user's ears in location and each forms a pocket or a cavity for receiving an inner space 120. Each one of the at least one slot 1211 is formed to allow access to at least one of the inner spaces 120. In this embodiment, the second headband 12 defines two slots 1211 and each slot 1211 communicates with a respective one of the corresponding inner spaces 120. Each slot 1211 can be formed to extend horizontally along a horizontal axis generally defined by the sleep mask when fully extended. This defines a length of the slot 1211 and a width W1 is defined perpendicular to the axis of the length of the slot 1211. The width W1 of each slot 1211 is sized and shaped to be smaller than a width defined by the corresponding inner space 120.

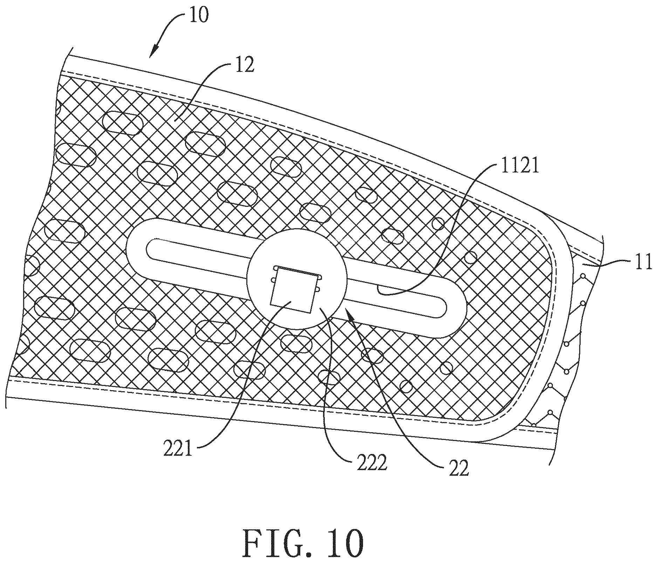

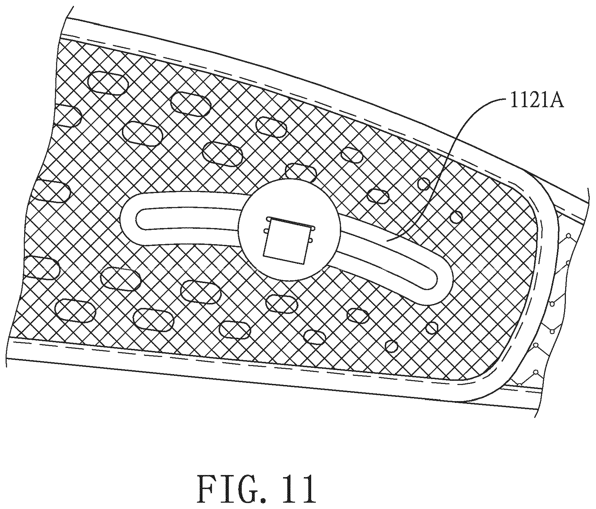

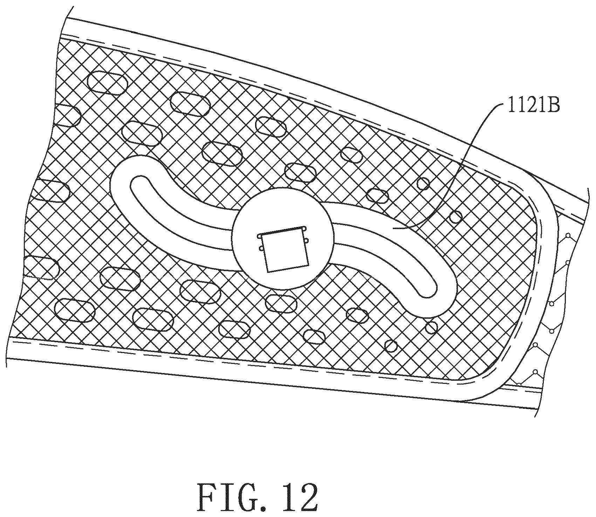

In present configuration shown in Fig. 10, each one of the slots 114 is straight, but it is not limited thereto. As shown in Fig. 11, the slot 1211A may be curved, and as shown in Fig. 12, the slot 1211B may be undulating or wavy, such that the speaker assemblies 20 can be adjusted and moved along the slot 1211A or slot 1211B in two directions.

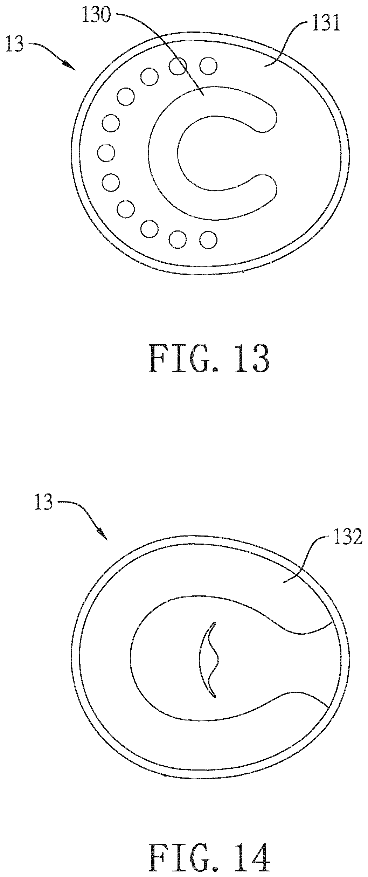

Please refer to Figs. 2, 13, and 14. The two eye cups 13 are configured to fit contours of eye sockets and cover the user's eyes, and thereby the eye cups 13 shade light thoroughly and provide better wearing experience. Eye cups 13 include a comfort pad that substantially surrounds an eye socket and defines an open pocket that provides space for a user's eye. This pocket configuration makes blinking and resting more comfortable as the surface of the eye cup generally avoids direct contact with the user's eyelid and/or eyelashes.

The two eye cups 13 can be detachably mounted on the first headband 11 and configured to cover the user's eyes respectively. Each one of the eye cups 13 has a front side 131 and a rear side 132 opposite each other. The rear side 132 is configured to cover one of the user's eyes. Each one of the eye cups 13 comprises an attaching portion 130. The attaching portion 130 of the eye cup 13 is mounted on the front side 131 of the eye cup 13 and configured to detachably attach onto the rear fluff layer 1121 of the first headband 11, such that the eye cup 13 is secured on the first headband 11. The eye cups 13 can be made of fabric, so the eye cups 13 are configured to be comfortable and breathable and are suitable for absorbing at least some of the user's sweat. Moreover, the eye cups 13 can be configured to be washable.

In another embodiment, the eye mask may not have eye cups 13, so the user's eyes are shaded by the first headband 11.

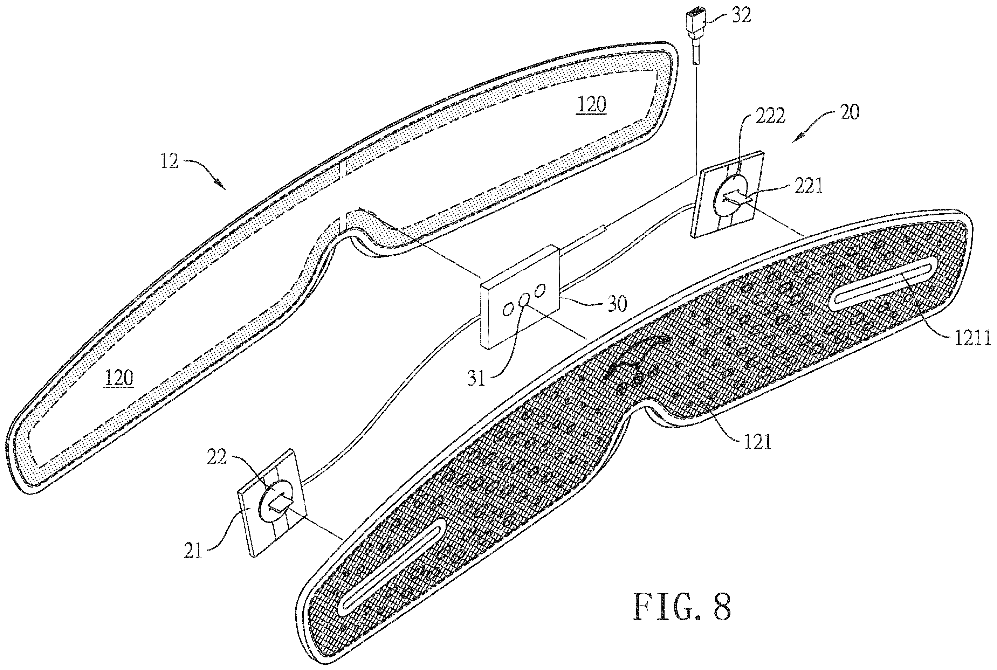

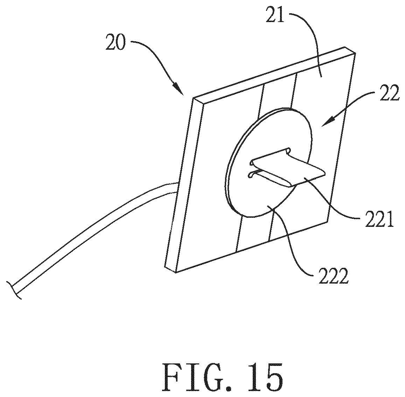

Please refer to Figs. 8 to 10 and 15. The two speaker assemblies 20 are provided in the inner spaces 120 respectively. At least one of the speaker assemblies 20 is configured to be manually moved or adjusted within the corresponding inner space 120. In an example, one of the speaker assemblies 20 is adjustable in location, but the other one may not.

In an example, in use, the user may adjust the entire eye mask so that the inner space 120 that cannot be adjusted is aligned with one ear, and then adjust the movable inner space 120 with respect to the entire eye mask to align with the other ear. In another embodiment, the two speaker assemblies 20 are movable and adjustable within the corresponding inner spaces 120.

The speaker assemblies 20 each include a speaker 21 and an operation unit 22. The speaker 21 is configured to be loose or floating within the pocket formed by inner space 120 and movably positioned within one of the inner spaces 120 of the mask main body 10. A general size of the speaker 21 is larger than that of the at least one slot 1211, such that the speaker 21 may not easily be moved from or unintentionally fall out of the inner space 120. Precisely, a width W2 of the speaker 21 is larger than the width W1 of the at least one slot 1211.

The operation unit 22 is located out of the mask main body 10 and connected to the speaker 21 via the at least one slot 1211, which allows the user to manually adjust or manipulate the operation unit 22 to slide or move the connected speaker 21. The operation unit 22 includes a tab 221 and selectively has a positioning piece 222. The tab 221 has two ends. One of the ends of the tab 221 is connected to the speaker 21 and the other end extends out from the slot 1211. The positioning piece 222 is mounted on the tab 221 and located between the two ends of the tab 221. A width W3 of the positioning piece 222 is larger than the width W1 of the at least one slot 1211.

The speaker assemblies 20 can be configured to be coupled to a hidden control unit 30 having multiple button 31, e.g. a power-on button, a connect button, and a volume control button. The speaker assemblies 20 can be coupled to the control unit 30 which is coupled to a power supply like a battery in the control unit 30. In this example, the power supply is a rechargeable battery that further includes an effectively concealed charging port 32. The speaker assemblies 20 are further coupled to the control unit 30 through hidden wires concealed within the second headband 12. It is further noted that the control unit 30 hidden wire and/or the speaker assemblies 20 are wirelessly accessible to allow the playing of sound or music from a wireless source like a music or noise app of a smartphone, tablet or otherwise. A wireless communication protocol or module can be incorporated into the control unit 30 to allow for BLUETOOTH compatibility or otherwise.

Please refer to Fig. 11 and Fig. 11. The eye mask in accordance with another embodiment of the present disclosure is provided. In this embodiment, the first headband 11C is a loop such that the first headband 11C may be secured on the user's head without any attach pad, which makes the entire first headband 11C tender and provides the user better experience. The loop-shaped first headband 11C is washable and breathable and is capable of absorbing sweat. Besides, the entire first headband 11C may be made of elastic material, an elastic ribbon for example, or only a section of the first headband 11C is made of elastic material. The eye mask may still have a second headband 12 detachably mounted on the first headband 11C and two speaker assemblies 20 mounted in the second headband 12. When wearing the eye mask, the user can envelope head in the first headband 11C arbitrarily, and then attach the second headband 12 on the first headband 11C according to the position of eyes and ears. Or, the user's head can be enveloped by both the first headband 11C and the second headband 12, and then adjust the first headband 11C until the second headband 12 aligning the position of eyes and ears.

With the aforementioned structures, the eye mask of the present disclosure is capable of not only shading light but also substantially reducing or blocking noise. When wearing the present eye mask, the user may cover the eyes and connect and tie two ends of the first headband 11, thereby securing the eye mask on the head. The eye mask also substantially covers the user's ears. Then, the two speaker assemblies 20 may be moved or adjusted along the two slots 1211 of the second headband 12 so that the two speaker assemblies 20 can align with the user's ears respectively. As a result, when the user desires some assistance to fall asleep, the two speaker assemblies 20 can provide a desired sound, music, or white noise to the user's ears, which can reduce or substantially block surrounding or environment noise and thus helps the user to sleep.

Besides, since the eye mask of the present disclosure includes a first headband 11 and the eye cups 13, and the first headband 11 and the eye cups 13 are configured to contact the user. Therefore, after putting on the present eye mask, the user may separate the first headband 11, the second headband 12, and the eye cups 13, and then only wash the first headband 11 and the eye cups 13 that are no longer clean or have absorbed the user's sweat. As a result, with the eye mask of the present disclosure, the user can wash the dirty part of the eye mask but still keep the speaker assemblies 20 intact.

## Claims

1. An eye mask comprising:
a mask main body (10) configured to cover a user's eyes and ears; the mask main body (10) including:
at least one inner space (120) corresponding to the user's ears in location; and
at least one slot (1211) formed to allow access to the at least one inner space (120);
at least one speaker assembly (20) mounted in the at least one inner space (120) and configured to be adjustable and movable in the at least one inner space (120); each one of the at least one speaker assembly (20) including:
a speaker (21) movably positioned in the at least one inner space (120) of the mask main body (10); and
an operation unit (22) located out of the mask main body (10) and connected to the speaker (21) via the at least one slot (1211), which allows the user to manually adjust or manipulate the operation unit (22) to slide or move the speaker (21);
the eye mask **characterized in that** the operation unit (22) includes:
a tab (221) having two ends, one of the two ends mounted on the speaker (21) and the other end extending out of the at least one slot (1211).

2. The eye mask as claimed in claim 1, wherein:
a width (W1) of the at least one slot (1211) is smaller than that of the at least one inner space (120); and
a width (W2) of the speaker (21) is larger than that of the at least one slot (1211).

3. The eye mask as claimed in claim 2, wherein:
the operation unit (22) includes:
a positioning piece (222) mounted on the tab (221) and located between the two ends of the tab (221), and a width of the positioning piece (222) being larger than that of the at least one slot (1211).

4. The eye mask as claimed in claim 1, wherein the at least one speaker assembly (20) includes two said speaker assemblies (20), the two speaker assemblies (20) are capable of moving in the at least one inner space (120).

5. The eye mask as claimed in claim 4, wherein the at least one slot (1211) includes two said slots (1211) so that the speaker assemblies can be aligned with the user's ears respectively.

6. The eye mask as claimed in any one of claims 1 to 5, wherein each one of the at least one slot (1211) is straight, curved, or undulating.

7. The eye mask as claimed in any one of claims 1 to 6 further comprising:
a control unit (30) mounted in the mask main body (10) and connected to the at least one speaker assembly (20), the control unit (30) including multiple buttons (31).

8. The eye mask as claimed in any one of claims 1 to 6, wherein the mask main body (10) includes:
a first headband (11) configured to contact the user;
a second headband (12) detachably mounted on the first headband (11), the at least one inner space (120) is formed in the second headband (12).

9. The eye mask as claimed in claim 8, wherein:
the mask main body (10) includes:
two eye cups (13) mounted on the first headband (11) and configured to cover the user's eyes respectively;
the first headband (11) has a front surface (111) and a rear surface (112) opposite each other, and the rear surface (112) faces the user; the first headband (11) comprises:
a fluff layer (1121) formed on the rear surface (112);
each one of the eye cups (13) has a front side and a rear side, the rear side is configured to cover one of the use's eyes; each one of the eye cups (13) comprises:
an attaching portion (130) mounted on the front side of the eye cup (13) and configured to detachably attach onto the fluff layer (1121), such that the eye cup (13) is detachably secured on the first headband (11).

10. The eye mask as claimed in claim 8, wherein the first headband (11) has a first end (113), a second end (114), and a middle section (115), the first end (113) and the second end (114) are opposite two ends of the first headband (11), the middle section (115) is between the first end (113) and the second end (114), and the middle section (115) is higher than the first end (113) and the second end (114) in location.

11. The eye mask as claimed in claim 8, wherein:
the first headband (11) has a first end (113) and a second end (114), the first end (113) and the second end (114) are opposite two ends of the first headband (11), the first end (113) has a front surface (111), the second end (114) has a rear surface (112), the front surface (111) of the first end (113) and the rear surface (112) of the second end (114) face inverse directions;
a fluff layer (1111) is formed on the front surface (111) of the first end (113); and
an attach pad (1122) is mounted on the rear surface (112) of the second end (114) and configured to detachably attach onto the fluff layer (1111), such that the second end (114) is detachably secured on the first end (113).

12. The eye mask as claimed in claim 8, wherein:
the first headband (11) has a front surface (111) and a rear surface (112) opposite each other, and the rear surface (112) faces the user; the first headband (11) comprises:
a fluff layer (1111) formed on the front surface (111);
the second headband (12) has a rear surface (122) facing the user and comprises:
an attaching portion (1221) mounted on the rear surface (122) of the second headband (12) and configured to detachably attach onto the fluff layer (1111), such that the second headband (12) is detachably secured on the first headband (11).

13. The eye mask as claimed in claim 8, wherein the first headband (11) is a strip or a closed loop.

## Patentansprüche

1. - Augenmaske, umfassend:
einen Maskenhauptkörper (10), der konfiguriert ist, um Augen und Ohren eines Benutzers zu bedecken; wobei der Maskenhauptkörper (10) Folgendes beinhaltet:
mindestens einen Innenraum (120), der der Position der Ohren des Benutzers entspricht; und
mindestens einen Schlitz (1211), der gebildet ist, um Zugang zu dem mindestens einen Innenraum (120) zu ermöglichen;
mindestens eine Lautsprecheranordnung (20), die in dem mindestens einen Innenraum (120) montiert und konfiguriert ist, um in dem mindestens einen Innenraum (120) einstellbar und bewegbar zu sein; wobei jede der mindestens einen Lautsprecheranordnung (20) Folgendes umfasst:
einen Lautsprecher (21), der bewegbar in dem mindestens einen Innenraum (120) des Maskenhauptkörpers (10) positioniert ist; und
eine Bedieneinheit (22), die sich außerhalb des Maskenhauptkörpers (10) befindet und über den mindestens einen Schlitz (1211), der es dem Benutzer ermöglicht, die Bedieneinheit (22) manuell einzustellen oder zu manipulieren, um den Lautsprecher (21) zu verschieben oder zu bewegen, mit dem Lautsprecher (21) verbunden ist;
wobei die Augenmaske, **dadurch gekennzeichnet, dass** die Bedieneinheit (22) Folgendes beinhaltet:
eine Lasche (221), die zwei Enden aufweist, wobei eines der zwei Enden an dem Lautsprecher (21) montiert ist und sich das andere Ende aus dem mindestens einen Schlitz (1211) erstreckt.

2. - Augenmaske nach Anspruch 1, wobei:
eine Breite (WI) des mindestens einen Schlitzes (1211) kleiner ist als die des mindestens einen Innenraums (120); und
eine Breite (W2) des Lautsprechers (21) größer ist als die des mindestens einen Schlitzes (1211).

3. - Augenmaske nach Anspruch 2, wobei:
die Bedieneinheit (22) Folgendes umfasst:
ein Positionierungsteil (222), das an der Lasche (221) montiert und sich zwischen den zwei Enden der Lasche (221) befindet, und wobei eine Breite des Positionierungsteils (222) größer ist als die des mindestens einen Schlitzes (1211).

4. - Augenmaske nach Anspruch 1, wobei die mindestens eine Lautsprecheranordnung (20) zwei dieser Lautsprecheranordnungen (20) beinhaltet, die zwei Lautsprecheranordnungen (20) in der Lage sind, sich in dem mindestens einen Innenraum (120) zu bewegen.

5. - Augenmaske nach Anspruch 4, wobei der mindestens eine Schlitz (1211) zwei der Schlitze (1211) beinhaltet, sodass die Lautsprecheranordnungen jeweils auf die Ohren des Benutzers ausgerichtet werden können.

6. - Augenmaske nach einem der Ansprüche 1 bis 5, wobei jeder von dem mindestens einen Schlitz (1211) gerade, gekrümmt oder gewellt ist.

7. - Augenmaske nach einem der Ansprüche 1 bis 6, ferner umfassend:
eine Steuereinheit (30), die in dem Maskenhauptkörper (10) montiert und mit der mindestens einen Lautsprecheranordnung (20) verbunden ist, wobei die Steuereinheit (30) mehrere Tasten (31) beinhaltet.

8. - Augenmaske nach einem der Ansprüche 1 bis 6, wobei der Maskenhauptkörper (10) Folgendes beinhaltet:
ein erstes Kopfband (11), das konfiguriert ist, um den Benutzer zu berühren;
ein zweites Kopfband (12), das abnehmbar an dem ersten Kopfband (11) montiert ist, wobei der mindestens eine Innenraum (120) in dem zweiten Kopfband (12) gebildet ist.

9. - Augenmaske nach Anspruch 8, wobei:
der Maskenhauptkörper (10) Folgendes beinhaltet:
zwei Augenschalen (13), die an dem ersten Kopfband (11) montiert und konfiguriert sind, um jeweils die Augen des Benutzers zu bedecken;
das erste Kopfband (11) eine vordere Fläche (111) und eine hintere Fläche (112) einander gegenüber aufweist, wobei die hintere Fläche (112) dem Benutzer zugewandt ist; wobei das erste Kopfband (11) Folgendes umfasst:
eine Flusenschicht (1121), die auf der hinteren Fläche (112) gebildet ist;
wobei jede der Augenschalen (13) eine Vorderseite und eine Rückseite aufweist, wobei die Rückseite konfiguriert ist, um eines der Augen des Benutzers zu bedecken; jede der Augenschalen (13) umfassend:
einen Anbringungsabschnitt (130), der an der Vorderseite der Augenschale (13) montiert und konfiguriert ist, um abnehmbar an der Flusenschicht (1121) angebracht zu werden, sodass die Augenschale (13) abnehmbar am ersten Kopfband (11) befestigt ist.

10. - Augenmaske nach Anspruch 8, wobei das erste Kopfband (11) ein erstes Ende (113), ein zweites Ende (114) und einen mittleren Abschnitt (115) aufweist, das erste Ende (113) und das zweite Ende (114) zwei gegenüberliegende Enden des ersten Kopfbands (11) sind, der mittlere Abschnitt (115) zwischen dem ersten Ende (113) und dem zweiten Ende (114) ist, und der mittlere Abschnitt (115) höher ist als das erste Ende (113) und das zweite Ende (114) am Standort.

11. - Augenmaske nach Anspruch 8, wobei:
das erste Kopfband (11) ein erstes Ende (113) und ein zweites Ende (114) aufweist, das erste Ende (113) und das zweite Ende (114) zwei gegenüberliegende Enden des ersten Kopfbands (11) sind, das erste Ende (113) eine vordere Fläche (111) aufweist, das zweite Ende (114) eine hintere Fläche (112) aufweist, die vordere Fläche (111) des ersten Endes (113) und die hintere Fläche (112) des zweiten Endes (114) in entgegengesetzte Richtungen gewandt sind;
eine Flusenschicht (1111) auf der Vorderfläche (111) des ersten Endes (113) gebildet ist; und
ein Befestigungspolster (1122) an der hinteren Fläche (112) des zweiten Endes (114) montiert und konfiguriert ist, um abnehmbar an der Flusenschicht (1111) angebracht zu sein, sodass das zweite Ende (114) abnehmbar an dem ersten Ende (113) angebracht ist.

12. - Augenmaske nach Anspruch 8, wobei:
das erste Kopfband (11) eine vordere Fläche (111) und eine hintere Fläche (112) einander gegenüber aufweist, wobei die hintere Fläche (112) dem Benutzer zugewandt ist; wobei das erste Kopfband (11) Folgendes umfasst:
eine Flusenschicht (1111), die auf der vorderen Fläche (111) gebildet ist;
das zweite Kopfband (12) eine Rückseite (122) aufweist, die dem Benutzer zugewandt ist und Folgendes umfasst:
einen Befestigungsabschnitt (1221), der an der hinteren Fläche (122) des zweiten Kopfbands (12) montiert und konfiguriert ist, um abnehmbar an der Flusenschicht (1111) angebracht zu sein, sodass das zweite Kopfband (12) abnehmbar an dem ersten Kopfband (11) befestigt ist.

13. - Augenmaske nach Anspruch 8, wobei das erste Kopfband (11) ein Streifen oder eine geschlossene Schlaufe ist.

## Revendications

1. - Masque pour les yeux comprenant:
un corps principal de masque (10) configuré pour recouvrir les yeux et les oreilles d'un utilisateur, le corps principal de masque (10) comprenant :
au moins un espace interne (120) correspondant en position aux oreilles de l'utilisateur ; et
au moins une fente (1211) formée pour permettre un accès à l'au moins un espace interne (120) ;
au moins un ensemble haut-parleur (20) monté dans l'au moins un espace interne (120) et configuré pour être réglable et déplaçable dans l'au moins un espace interne (120) ; chacun de l'au moins un ensemble haut-parleur (20) comprenant :
un haut-parleur (21) positionné de manière mobile dans l'au moins un espace interne (120) du corps principal de masque (10) ; et
une unité d'actionnement (22) située à l'extérieur du corps principal de masque (10) et reliée au haut-parleur (21) par l'intermédiaire de l'au moins une fente (1211), ce qui permet à l'utilisateur de régler ou manipuler manuellement l'unité d'actionnement (22) pour faire glisser ou déplacer le haut-parleur (21) ;
le masque pour les yeux étant **caractérisé par le fait que** l'unité d'actionnement (22) comprend :
une languette (221) ayant deux extrémités, l'une des deux extrémités étant montée sur le haut-parleur (21) et l'autre extrémité s'étendant hors de l'au moins une fente (1211).

2. - Masque pour les yeux selon la revendication 1, dans lequel :
une largeur (W1) de l'au moins une fente (1211) est inférieure à celle de l'au moins un espace interne (120) ; et
une largeur (W2) du haut-parleur (21) est supérieure à celle de l'au moins une fente (1211).

3. - Masque pour les yeux selon la revendication 2, dans lequel :
l'unité d'actionnement (22) comprend :
une pièce de positionnement (222) montée sur la languette (221) et située entre les deux extrémités de la languette (221), et une largeur de la pièce de positionnement (222) étant supérieure à celle de l'au moins une fente (1211).

4. - Masque pour les yeux selon la revendication 1, dans lequel l'au moins un ensemble haut-parleur (20) comprend deux ensembles haut-parleurs (20) précités, les deux ensembles haut-parleurs (20) étant aptes à se déplacer dans l'au moins un espace interne (120).

5. - Masque pour les yeux selon la revendication 4, dans lequel l'au moins une fente (1211) comprend deux fentes (1211) précitées, de telle sorte que les ensembles haut-parleurs peuvent être alignés respectivement avec les oreilles de l'utilisateur.

6. - Masque pour les yeux selon l'une quelconque des revendications 1 à 5, dans lequel chacune de l'au moins une fente (1211) est rectiligne, incurvée ou ondulée.

7. - Masque pour les yeux selon l'une quelconque des revendications 1 à 6, comprenant en outre :
une unité de commande (30) montée dans le corps principal de masque (10) et connectée à l'au moins un ensemble haut-parleur (20), l'unité de commande (30) comprenant plusieurs boutons (31).

8. - Masque pour les yeux selon l'une quelconque des revendications 1 à 6, dans lequel le corps principal de masque (10) comprend :
un premier bandeau de tête (11) configuré pour être en contact avec l'utilisateur ;
un second bandeau de tête (12) monté de manière détachable sur le premier bandeau de tête (11), l'au moins un espace interne (120) étant formé dans le second bandeau de tête (12).

9. - Masque pour les yeux selon la revendication 8, dans lequel :
le corps principal de masque (10) comprend :
deux coques oculaires (13) montées sur le premier bandeau de tête (11) et configurées pour recouvrir respectivement les yeux de l'utilisateur ;
le premier bandeau de tête (11) a une surface avant (111) et une surface arrière (112) opposées l'une à l'autre, et la surface arrière (112) fait face à l'utilisateur, le premier bandeau de tête (11) comprend :
une couche de duvet (1121) formée sur la surface arrière (112) ;
chacune des coques oculaires (13) a un côté avant et un côté arrière, le côté arrière est configuré pour recouvrir l'un des yeux de l'utilisateur ; chacune des coques oculaires (13) comprend :
une partie de fixation (130) montée sur le côté avant de la coque oculaire (13) et configurée pour se fixer de manière détachable sur la couche de duvet (1121), de telle sorte que la coque oculaire (13) est fixée de manière détachable sur le premier bandeau de tête (11).

10. - Masque pour les yeux selon la revendication 8, dans lequel le premier bandeau de tête (11) a une première extrémité (113), une seconde extrémité (114) et une section centrale (115), la première extrémité (113) et la seconde extrémité (114) sont deux extrémités opposées du premier bandeau de tête (11), la section centrale (115) est située entre la première extrémité (113) et la seconde extrémité (114), et la section centrale (115) est plus haute que la première extrémité (113) et la seconde extrémité (114) en position.

11. - Masque pour les yeux selon la revendication 8, dans lequel :
le premier bandeau de tête (11) a une première extrémité (113) et une seconde extrémité (114), la première extrémité (113) et la seconde extrémité (114) sont deux extrémités opposées du premier bandeau de tête (11), la première extrémité (113) a une surface avant (111), la seconde extrémité (114) a une surface arrière (112), la surface avant (111) de la première extrémité (113) et la surface arrière (112) de la seconde extrémité (114) sont orientées dans des directions inverses ;
une couche de duvet (1111) est formée sur la surface avant (111) de la première extrémité (113) ; et
un tampon de fixation (1122) est monté sur la surface arrière (112) de la seconde extrémité (114) et configuré pour se fixer de manière détachable sur la couche de duvet (1111), de telle sorte que la seconde extrémité (114) est fixée de manière détachable sur la première extrémité (113).

12. - Masque pour les yeux selon la revendication 8, dans lequel :
le premier bandeau de tête (11) a une surface avant (111) et une surface arrière (112) opposées l'une à l'autre, et la surface arrière (112) fait face à l'utilisateur ; le premier bandeau de tête (11) comprend :
une couche de duvet (1111) formée sur la surface avant (111) ;
le second bandeau de tête (12) a une surface arrière (122) faisant face à l'utilisateur et comprend :
une partie de fixation (1221) montée sur la surface arrière (122) du second bandeau de tête (12) et configurée pour se fixer de manière détachable sur la couche de duvet (1111), de telle sorte que le second bandeau de tête (12) est fixé de manière détachable sur le premier bandeau de tête (11).

13. - Masque pour les yeux selon la revendication 8, dans lequel le premier bandeau de tête (11) est une bande ou une boucle fermée.
